# EUROPÄISCHE PATENTANMELDUNG

(11) **EP 1 407 765 A1**
(43) Veröffentlichungstag der Anmeldung: **14.04.2004**
(21) Anmeldenummer: 03022713.6
(22) Anmeldetag: 09.10.2003
(51) Int. Cl.: A61K 9/127, A61K 9/107, A61K 39/39, A61K 47/48

(54) **Vesikel mit chimären Rezeptoren zur Induktion einer gerichteten T-Zellantwort in vivo**

(30) Priorität: 10.10.2002 DE 10247256; 15.02.2003 EP 03003624
(71) Anmelder: Fraunhofer-Gesellschaft zur Förderung der angewandten Forschung e.V., 80686 München (DE)
(72) Erfinder: Barth, Stefan, Dr. Dr., Worringer Strasse 1 52074 Aachen (DE); Finnern, Ricarda, 52070 Aachen (DE); Klockenbring, Torsten, 53121 Bonn (DE); Fischer, Rainer, 52156 Monschau (DE)
(74) Vertreter: Schreiber, Christoph, Dr.

(57) **Zusammenfassung**

Vesikel mit einem inneren Kompartiment, einer Membran und einer Außenseite, wobei das Vesikel an der Außenseite mindestens ein Molekül trägt mit einer ersten Domäne, die mindestens eine Bindungsstelle für Dendritische Zellen, Langerhans-Zellen, Makrophagen/Monozyten, Enterozyten, M-Zellen und B-Lymphozyten und ein weiteres Antigen in der Membran des Vesikels verankert ist und einer zweiten Domäne, die eine membranständige Region ist, und das Bindungsmolekül in der Membran des Vesikels verankert.

## Beschreibung

Die vorliegende Erfindung betrifft Vesikel mit chimären Rezeptoren und ihre Verwendung.

Zur Aktivierung einer Immunantwort durch T-Lymphozyten auf ein Pathogen (Antigen) muss das Antigen im Verbund mit dem Major-Histocompatibilitätskomplex (MHC) den T-Lymphozyten präsentiert werden. Diese Aufgabe wird von antigenpräsentierenden Zellen, den sogenannten APCs, übernommen. Sie vermitteln zwischen der humoralen (Antikörper) und der zellulären Immunantwort. Für das Zusammenspiel zwischen antigenpräsentierenden Zellen und T-Lymphozyten ist das Vorhandensein bestimmter Adhäsionsmoleküle (ICAM-1, ICAM-2, LFA-1 und LFA-3) und costimulierender Rezeptoren, wie die B7.1(CD80)- und B7.2(CD86)- Moleküle, auf der Zelloberfläche von Bedeutung. Diese Voraussetzung wird in erster Linie sowohl von Dendritischen Zellen, den Langerhans-Zellen als auch von Makrophagen und B-Lymphozyten erfüllt. Von einigen antigenpräsentierenden Zellen werden die MHC-II-Moleküle dauernd exprimiert, weshalb sie auch als konstitutiv antigenpräsentierende Zellen bezeichnet werden. Dazu gehören interdigitierende Dendritische Zellen der lymphatischen Gewebe, Langerhans-Zellen, B-Lymphozyten, M-Zellen, aktivierte Monozyten bzw. Makrophagen und 15% der ruhenden Makrophagen. Fakultativ antigenpräsentierende Zellen, wie z.B. follikuläre Zellen der Schilddrüse, Endothelzellen und Fibroblasten, werden erst durch Cytokine (z.B. IFN-γ) zur Exprimierung der MHC-II-Moleküle angeregt.

Aufgabe der Erfindung ist es, Vesikel bereitzustellen, die so modifiziert sind, dass mit ihnen eine gerichtete T-Zellantwort ausgelöst werden kann.

Gelöst wird die Aufgabe durch ein Vesikel mit einem inneren Kompartiment, einer Membran und einer Außenseite, wobei das Vesikel an der Außenseite mindestens ein Bindungsmolekül trägt mit einer ersten Domäne, die mindestens eine Bindungsstelle für Dendritische Zellen, Langerhans-Zellen, Makrophagen/Monozyten, Enterozyten, M-Zellen und B-Lymphozyten und ein weiteres Antigen in der Membran des Vesikels verankert ist und einer zweiten Domäne, die eine membranständige Region ist, und das Bindungsmolekül in der Membran des Vesikels verankert. Das innere Kompartiment kann z.B. Antigen, DNA oder therapeutische Substanzen enthalten.

Das Vesikel umfasst also ein Kompartiment, das zum Beispiel Antigene enthalten kann. Das innere Kompartiment ist von einer Membran umschlossen. In dieser Membran befindet sich ein Bindungsmolekül, das beispielsweise ein Fusionsmolekül aus zwei bekannten Proteinen ist. Ein Teil des Bindungsmolekül, die erste Domäne, weist dabei eine Bindungsstelle für die genannten Zellen auf. Ein zweiter Teil des Bindungsmoleküls, die zweite Domäne, ist membranständig und verankert somit das Bindungsmolekül in der Membran des Vesikels, während die erste Domäne nach außen, weg vom inneren Kompartiment gerichtet ist.

In einer bevorzugten Ausführungsform weist das Vesikel ein weiteres Antigen auf, das sich von dem Bindungsmolekül unterscheidet. Das Antigen kann sowohl an der Membran außen als auch an der Innenseite verankert sein.

Wenn das Vesikel über die erste Domäne an Dendritische Zellen, Langerhans-Zellen, Makrophagen/Monozyten, Enterozyten, M-Zellen und B-Lymphozyten bindet, führt dies zu einer Internalisierung des Vesikels. Da das Vesikel wesentlich mehr Antigene enthalten kann als üblicherweise durch die sonst internalisierten Antigen/Antikörper-Komplexe erreicht wird, lässt sich auf diesem Wege eine Immunantwort initiieren und/oder verstärken oder auch abschwächen und/oder unterdrücken. Diese erfolgte Aufnahme bewirkt eine Aktivierung der Dendritischen Zellen, Langerhans-Zellen, Makrophagen/Monozyten und B-Lymphozyten.

Die erste Domäne ist eine Bindungsstelle, die für Dendritische Zellen, Langerhans-Zellen, Makrophagen/Monozyten, Enterozyten, M-Zellen und B-Lymphozyten selektiv ist. Spezifische Oberflächenmarker sind teilweise bekannt. Dies sind beispielsweise CD64-, CD80-, CD86-, CD89-, CD11c, MHC II-Rezeptor oder nicht polymorphe MHC I ähnliche Moleküle CD 1a-d [Präsentation von Lipiden und Glycolipiden bakterieller Herkunft auf Epithelzellen, z.B. Enterozyten].

Die erste Domäne kann deshalb ein rekombinanter Ligand mit Spezifität gegen mindestens einen der Oberflächenmarker sein, beispielsweise Immunglobulin-Fc-Teile (sowohl IgG, als auch IgA), einzelsträngige Antikörperfragmente (scFv), Fab, (Fab')₂, Bindungspeptide deren rekombinante natürliche Liganden (bspw. CD28, CTLA4) oder andere artifizielle Bindungsstrukturen; möglich wären aber auch Superantigene oder Toll-like Rezeptorliganden.

Eine ebenfalls vorhandene erste Domäne ist ein peptidisches Antigen, bzw. seine Mutationen, Modifikationen, homologen poly-Epitopvarianten. Diese Domäne kann sowohl extrazellulär als auch intrazellulär exprimiert sein. Als Vesikel können auch komplette Zellen eingesetzt werden.

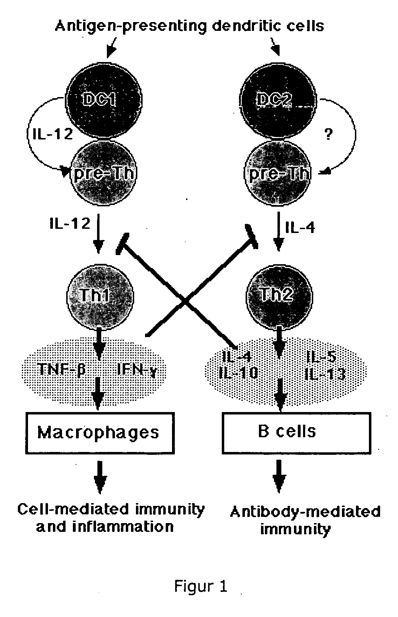

Das Kompartiment enthält oder trägt - neben den an der Außenmembran assoziierten Antigenen - bevorzugt Antigene, insbesondere peptidische Antigene oder korrespondierende rekombinante DNA. Die Antigene können beispielsweise peptidische Antigene von Humanpathogenen (z.B. Chlamydien, Salmonellen, Mykobakterien, LCMV, Listerien, Shigellen, Rickettsien, akute und chronische LCMV, HIV, HCV, HBV, EBV, RSV, MHV, HTLV, HSV, CMV, Adeno, Herpes-, Sendai-, Vaccinia-, Pavo-, Influenza-Viren, Ectromelia), HSP 70 heat shock Vakzinierungspeptide oder spezifische Tumorantigene, idiotypische Antikörper (-fragmente), anti idiotypische, Epitop-mimikrierende Antikörper, mutierte Autoantigene (bspw. EGF-Rezeptor, ein MUC-1-Antigen mit verändertem Glycosylierungsmuster) oder spezifische Botenstoffe sein. Alternativ kann es sich auch um in Eukaryonten translatierbare rekombinante DNA handeln, die beispielsweise für Antigene von Humanpathogenen wie Bakterien, Viren, Helminthen, Pilzen, Prionen oder spezifische Tumorantigene, idiotypische Antikörper (-fragmente) oder mutierte Autoantigene kodiert. Je nach Antigen kann eine Th1- oder Th2-Antwort induziert oder unterdrückt werden (Figur 1).

Die erfindungsgemäßen Vesikel eignen sich insbesondere zur Vakzinierung: Eine Th-1 Antwort lässt sich durch Verwendung von Antigenplasmiden in Kombination mit bakteriellen, immunstimmulatorischen Sequenzen (CpG-Motive) erzielen. Dabei werden die auf den Plasmiden kodierten antigenen Determinanten nach ihrer Internalisierung synthetisiert und gelangen analog zu viralen Elementen im MHC I-Kontext zur Präsentation. Es werden vornehmlich Th1-dominierte Immunreaktionen initialisiert, die eine zusätzliche Verstärkung durch CpG-Motive erhalten. Obwohl der zugrunde liegende Wirkmechanismus noch nicht hinreichend aufgeklärt werden konnte, scheinen CpG-Motive dem Immunsystem eine bakterielle Infektion zu signalisieren, die Th1-vermittelte Immunreaktionen hervorrufen. Dies ist für das Mausmodell belegt und wird am Menschen derzeit auf der Ebene klinischer Studien der Phase I untersucht. Die Entwicklung von Vakzinierungsverfahren zur gezielten Induktion Th2-dominierter Immunreaktionen ist Gegenstand intensiver Forschungstätigkeit z.B. im Rahmen der Malariaprophylaxe oder der Therapie von *Heliobacter pylori*-Infektionen. Formalin-fixierte respiratory syncytical (RS) Viren induzieren starke Th2, IL4 und IL5 dominierte, Immunantworten mit ausgeprägter Rekrutierung eosinophiler Granulozyten. Adenovirus transfizierte Dendritische Zellen, die macrophage-derived Chemokine (MDC) überexprimieren, konnten nach Vakzinierung mit hitzeinaktivierten *Pseudomonas aeruginosa* protektive Th2 dominierte Immunreaktionen im Mausmodell induzieren. Im Mausmodell ist es ebenfalls möglich Dendritische Zellen ,ex vivo' mit chlamydial major outer membrane protein (rMOMP) zu pulsen. Diese Zellen rufen - reinjiziert - *in vivo* Th2 Antworten gegen rMOMP hervor.

Das Vesikel kann in der einen Ausführungsform ein Liposom, eine Mizelle oder ein Aerosoltröpfchen sein, in das Bindungsmoleküle eingelagert sind. Alternativ kann es sich um eine nicht-Säugerzelle handeln, wobei in diesem Fall bevorzugt das Bindungsmolekül direkt von der Zelle exprimiert wird und transmembranständig angeordnet wird.

Solche nicht-Säugerzellen können sein: Bakterien, Hefen, Viren, (Bakterio-) Phagen, Pflanzenzellen, Algen, Zellorganellen.

Erhältlich sind entsprechende Vesikel durch gängige Verfahren nach Stand der Technik: Immunoliposomen als Träger von Zytostatika (Li et al., Cancer Gene Ther, 2001 Aug, 8(8): 555-65; Park et al., J Control Release, 2001 Jul 6, 74(1-3): 95-113; Gagne et al., BBA, 2002 Feb, 1558: 198-210; Mastrobattista et al., Adv Drug Deliv Rev 1999 Nov 10, 40(1-2): 103-127), Oilbodies (Chen & Tzen, Plant Cell Physiol 2001 Nov, 42(11): 1245-52; Abell et al., J Biol Chem 2002 Mar 8, 277(10): 8602-10; Giddings et al., Nat Biotechnol 2000 Nov; 18(11): 1151-5), Mizellen (Mastrobattista et al., Cancer Gene Ther 2001 Jun, 8(6): 405-13; Torchilin, J Control Release 2001 Jun 15, 73(2-3): 137-72; Rosler et al., Adv Drug Deliv Rev 2001 Dec 3, 53(1): 95-108).

Als Transmembrandomänen können beispielsweise die beta-, gamma, oder zeta-Kette des humanen T-Zellrezeptors (Rossig et al., Blood 2002 Mar 15, 99(6): 2009-16; Maher et al., Nat Biotechnol 2002 Jan, 20(1): 70-5; Hombach et al., Gene Ther 2001 Jun, 8(11): 891-5; Hombach et al., Cancer Res 2001 Mar 1, 61(5): 1976-82), Oberflächenproteine von Bakteriophagen (pIII, pVI, pVII, pVIII, pIX), Hüllproteine von Viren (beispielsweise Alfalfa) aber auch genetisch modifizierte Oleosine oder Caleosine aus Oilbodies dienen.

Die Erfindung wird durch die folgenden Beispiele näher erläutert.

### Beispiel 1

### Präparation von Liposomen

Zur Herstellung von Liposomen wird lyophilisiertes Pulver von E. coli polar total lipid extract (Avanti Polar Lipids, Inc., USA), L-α-Phosphatidylcholin, L-α-Phosphatidylglycerin (jeweils aus Eigelb; Sigma, Steinheim) oder Mischungen dieser Lipide in 100 mM Kpi (pH 7,5), 2 mM β-Mercaptoethanol unter Zugabe von 1,5% (w/v) n-Octyl-b-D-Glucopyra-nosid (Calbiochem, Bad Soden) gelöst und bei RT gerührt. Dabei wird eine Lipidkonzentration von 20 mg/ml eingestellt. Die Lipidsuspension wird in Dialyseschläuche (Durchmesser: 6 mm; Porengröße: 2,5 nm; Serva, Heidelberg) gefüllt und gegen das 20-fache Volumen 100 mM KPi (pH 7,5), 2 mM â-Mercaptoethanol unter leichtem Rühren bei RT dialysiert. Der Puffer wird dreimal nach jeweils 4-5 h gewechselt. Die dialysierten Lipide werden aliquotiert und bei -80°C gelagert.

### Trübungsmessung

1 ml Liposomen (circa 0,5 - 2 mg/ml Lipid) wird durch circa 10-maliges Extrudieren durch eine Polycarbonatmembran mit einer Porengröße von 400 nm (LFLM-400; Milsch, Laudenbach) zu unilaminaren Liposomen einheitlicher Größe geformt. Bei dem hierzu verwendeten Extruder handelte es sich um einen LiposoFast ™ -Basic von der Firma Avestin Inc. (Ottawa, Kanada). Zur Verhinderung der erneuten Ausbildung von multilaminaren Strukturen werden alle folgenden Schritte bei RT durchgeführt. Die optische Dichte der Liposomensuspension wird im Photometer bei 600 nm bestimmt. Durch sukzessiven Zusatz einer 20%-igen (v/v) Triton X-100-Lösung kann die zunehmende Solubilisierung der Liposomen bis hin zur vollständigen ,mixed micelle'-Bildung über die Abnahme der optischen Dichte verfolgt werden.

### Rekonstitution von Proteinen in Liposomen

Der Einbau von Proteinen in Liposomen erfolgt nach der Methode von Rigaud et al. (1995). Zur Rekonstitution des Proteins in Liposomen werden diese in 100 mM KPi (pH 7,5) auf eine Konzentration von 5 mg/ml verdünnt und circa zehnmal extrudiert (Porengröße: 400 nm). Die extrudierten Liposomen werden für 10 min unter leichtem Schütteln bei RT mit 0,5% Triton X-100 (v/v; entspricht 1 µl/mg Lipid) behandelt; dies führt zu einer 15%-igen Solubilisierung der Liposomen. Gereinigtes Strep-BetP wird mit den solubilisierten Liposomen gemischt, wobei, wenn nicht anders angegeben, ein Lipid-Protein-Verhältnis (LPR) von 30:1 (w/w) eingestellt wird. Zur Abreicherung des Detergenz wird direkt anschließend der Ansatz mit x mg Biobeads (Naßgewicht; BioRad, München; x = eingesetzte µl Triton X-100 * 5 + eingesetzte µg Dodecylmaltosid * 10) zweimal für je 1 h bei RT und einmal mit der doppelten Menge Biobeads über Nacht bei 4°C inkubiert, wodurch der Einbau des Proteins in die Liposomen gewährleistet wird. Nach Abtrennung der Biobeads werden die Proteoliposomen zentrifugiert (400.000 g, 20 min, 15°C), zweimal mit 100 mM KPi (pH 7,5) gewaschen, im gleichen Puffer resuspendiert (circa 400 µl/mg eingesetztes Protein), aliquotiert und bei -80°C gelagert.

### Beispiel 2

In ein nach Stand der Technik generiertes Liposom wird ein rekombinant exprimiertes anti-CD64-Bindungsmolekül mit Transmembrandomäne durch mehrstündige Inkubation bei RT inkorporiert. In das Liposom werden peptidische Fragmente von Shigellen eingebracht.

### Beispiel 3

Heliobacter pylori Vesikel zur Induktion einer Th2-Antwort mit chimärem Rezeptor durch den gleichzeitig eine Bindung an antigenpräsentierende Zellen ermöglicht wird und die heliobacter pylori Peptide dargestellt werden.

In eine nach Stand der Technik generierte Mizelle wird ein rekombinant exprimiertes, chimäres anti-MHCII-Bindungsmolekül gekoppelt mit Transmembrandomäne und Formalin-fixierten respiratory syncytical (RS) Viren durch mehrstündige Inkubation bei RT inkorporiert.

In ein nach Stand der Technik generiertes Liposom wird ein rekombinant exprimiertes, chimäres anti-MHCII-Bindungsmolekül gekoppelt mit Transmembrandomäne und Heliobacter Pylori-Peptid durch mehrstündige Inkubation bei RT inkorporiert. In das Liposom werden peptidische Formalin-fixierten respiratory syncytical (RS) Virus Fragmente eingebracht.

### Beispiel 4

### Vesikel zur Induktion einer Th1-Antwort gegen HCV

In ein nach Stand der Technik generiertes Liposom wird ein rekombinant exprimiertes, chimäres anti-MHCI-Bindungsmolekül mit Transmembrandomäne durch mehrstündige Inkubation bei RT inkorporiert. In das Liposom werden eukaryotische Expressionsplasmide mit kodierenden Regionen für HCV-Fragmente eingebracht.

### Beispiel 5

### Rekombinante Pflanzenviren mit parallel exprimierten chimären anti-CD89-Bindungsmolekülen und membranständigen HIV-Peptiden

Hüllproteine von Pflanzenviren (Alfalfa-Mosaikvirus) werden einerseits mit anti-CD89-Bindungsmolekülen und andererseits mit HIV-Peptiden fusioniert. Die aus den Pflanzen nach Stand der Technik isolierten Virenpartikel werden nach Aufreinigung unmittelbar zur Vakzinierung eingesetzt.

### Beispiel 6

### CD64-spezifische filamentöse Bakteriophagen zur Induktion einer Immunantwort gegen Herpesvirus

An der Oberfläche von filamentösen Bakteriophagen werden nach Stand der Technik Fusionen von anti-CD64 Antikörperfragmenten dargestellt. Das gepackte Phagemid enthält zusätzlich Herpesvirusfragmente kontrolliert von einem eukaryotischen Promoter. Eine zusätzliche Verstärkung kann durch CpG-Motive erhalten werden.

An der Oberfläche von filamentösen Bakteriophagen werden nach Stand der Technik Fusionen von anti-CD64 Antikörperfragmenten mit dem Oberflächenprotein pVI und von hsp 70 mit dem Oberflächenprotein pIX dargestellt. Das gepackte Phagemid enthält zusätzlich Herpesvirusfragmente kontrolliert von einem eukaryotischen Promoter.

### Beispiel 7

### Transgenes Bakterium mit membranständigem M-zellspezifischen Bindungsliganden und Multicopy-Plasmid zur eukaryotischen Expression von Influenza-Peptiden

Membranintegrierte M-zellspezifische Bindungsliganden werden nach Stand der Technik an der Oberfläche Gram-positiver Bakterien (Staphylococcus carnosus) dargestellt. Die Organismen sind transfomiert mit einem Multicopy-Plasmid mit kodierenden Regionen von Influenzaviren kontrolliert von einem eukaryotischen Promoter. Eine zusätzliche Verstärkung kann durch CpG-Motive erhalten werden.

### Beispiel 8

### Oilbodies zur Induktion einer Th2-Antwort gegen Malaria

Rekombinante Oleosine mit einem anti-CD64-Peptidmimotop am C-Terminus und einem Malariapeptid am N-Terminus werden durch Stand der Technik konstruiert und als Oilbodies aus Pflanzen isoliert.

Rekombinante Caleosine modifiziert durch ein anti-CD64-Peptidmimotop und rekombinante Oleosine modifiziert mit Malariapeptiden werden durch Stand der Technik konstruiert und als Oilbodies aus Pflanzen isoliert.

## Patentansprüche

1. Vesikel mit einem inneren Kompartiment, einer Membran und einer Außenseite, wobei das Vesikel an der Außenseite mindestens ein Molekül trägt mit einer ersten Domäne, die mindestens eine Bindungsstelle für Dendritische Zellen, Langerhans-Zellen, Makrophagen/Monozyten, Enterozyten, M-Zellen und B-Lymphozyten und ein weiteres Antigen in der Membran des Vesikels verankert ist und einer zweiten Domäne, die eine membranständige Region ist, und das Bindungsmolekül in der Membran des Vesikels verankert.

2. Vesikel nach Anspruch 1, **dadurch gekennzeichnet, dass** die Bindung an Dendritische Zellen, Langerhans-Zellen, Makrophagen/Monozyten und B-Lymphozyten eine Aktivierung der Dendritische Zellen, Langerhans-Zellen, Makrophagen/Monozyten und B-Lymphozyten bewirkt.

3. Vesikel nach Anspruch 1 und/oder 2, **dadurch gekennzeichnet, dass** die erste Domäne eine Bindungsstelle für CD16, CD32, CD64, CD35, FαRI, CD11b, CDw17, CD68, CD14, CD206, CD172a, MHC I, class II , CD204, CD206, MACRO-, CD80-, CD86-, CD89-, CD11c, MHC II-Rezeptor aufweist.

4. Vesikel nach einem der Ansprüche 1 bis 3, **dadurch gekennzeichnet, dass** die erste Domäne ein rekombinanter Ligand mit Spezifität gegen mindestens einen der Oberflächenmarker ist, beispielsweise Immunglobulin-Fc-Teile (sowohl IgG, als auch IgA), mono- und bivalente einzelsträngige Antikörperfragmente (scFv), Fab, (Fab')₂, Bindungspeptide, deren rekombinante natürliche Liganden, Toll-ähnliche Rezeptorliganden, andere artifizielle Bindungsstrukturen, Superantigene und Modifikationen oder Kombinationen davon.

5. Vesikel nach einem der Ansprüche 1 oder 4, **dadurch gekennzeichnet, dass** das Kompartiment peptidische Antigene oder deren korrespondierende rekombinante DNA enthält oder ebenfalls an der Membran trägt.

6. Vesikel nach Anspruch 5, **dadurch gekennzeichnet, dass** die Antigene abgeleitet sind aus peptidischen Antigenen von Humanpathogenen (z.B. Chlamydien, Salmonellen, Mykobaterien, LCMV) oder spezifischen Tumorantigenen, idiotypischen Antikörpern (-fragmente), mutierten Autoantigenen (bspw. EGF-Rezeptor, ein MUC-1-Antigen mit verändertem Glycosylierungsmuster) oder spezifischen Botenstoffen. Alternativ kann es sich auch um in Eukaryonten translatierbare rekombinante DNA handeln, die beispielsweise für Antigene von Humanpathogenen wie Bakterien, Viren, Helminthen, Pilzen, Prionen oder für spezifische Tumorantigene, idiotypische Antikörper (-fragmente) oder mutierte Autoantigene kodieren.

7. Vesikel nach einem der Ansprüche 1 bis 6, **dadurch gekennzeichnet, dass** über die angereicherten Antigene gezielt eine Th1- oder Th2-Antwort induziert oder unterdrückt werden kann.

8. Vesikel nach einem der Ansprüche 1 bis 7, **dadurch gekennzeichnet, dass** das Vesikel ein Liposom, eine Mizelle, ein Aerosol, Oilbodies oder eine nicht-Säugerzelle umfasst.

9. Nicht-Säugerzelle nach Anspruch 8, **dadurch gekennzeichnet, dass** das Bindungsmolekül von der Zelle exprimiert wird.

10. Nicht-Säugerzelle nach Anspruch 9, **dadurch gekennzeichnet, dass** die Zelle ausgewählt ist aus Bakterien, Hefen, Viren, (Bakterio-) Phagen, Pflanzenzellen, Algen, Zellorganellen.

11. Verfahren zur Herstellung der Vesikel nach einem der Ansprüche 1 bis 10, **dadurch gekennzeichnet, dass** Membranproteine in definierter Orientierung in Lipidmembranen von Liposomen integriert werden .

12. Verwendung der Vesikel nach einem der Ansprüche 1 bis 8 zur Vakzinierung.
